# EUROPEAN PATENT SPECIFICATION

(11) **EP 1 679 960 B1**
(45) Date of publication and mention of the grant of the patent: **06.05.2009**
(21) Application number: 04818159.8
(22) Date of filing: 08.11.2004
(51) Int. Cl.: A01K 67/033, A01K 67/027, A61K 49/00

(54) **TRANSGENIC AMPHIBIAN MODELS FOR LYMPHATIC VESSEL DEVELOPMENT**
TRANSGENE AMPHIBISCHE TIERMODELLE FÜR LYMPHANGIOGENESE
MODELES AMPHIBIENS TRANSGENIQUES DESTINES AU DEVELOPPEMENT DE VAISSEAUX LYMPHATIQUES

(30) Priority: 07.11.2003 US 517884 P; 07.11.2003 EP 03104123; 17.09.2004 EP 04077583; 22.09.2004 US 611705 P
(43) Date of publication of application: 19.07.2006
(73) Proprietor: VIB vzw, 9052 Zwijnaarde (BE); Life Sciences Research Partners VZW., 3000 Leuven (BE)
(72) Inventor: CARMELIET, Peter, B-3052 Blanden (BE)
(86) International application number: PCT/EP2004/052872
(87) International publication number: WO 2005/043993

(56) References cited:
- US-A1- 2001 037 016
- HONG YOUNG-KWON ET AL: "Prox1, master regulator of the lymphatic vasculature phenotype." CELL AND TISSUE RESEARCH. OCT 2003, vol. 314, no. 1, October 2003 (2003-10), pages 85-92, XP002319340 ISSN: 0302-766X
- DEL RIO-TSONIS KATIA ET AL: "Regulation of Prox 1 during lens regeneration" IOVS, vol. 40, no. 9, August 1999 (1999-08), pages 2039-2045, XP002319342
- ALITALO KARI ET AL: "Molecular mechanisms of lymphangiogenesis in health and disease" CANCER CELL, vol. 1, no. 3, April 2002 (2002-04), pages 219-227, XP002272726 ISSN: 1535-6108
- ROCKSON STANLEY G: "Preclinical models of lymphatic disease: the potential for growth factor and gene therapy." ANNALS OF THE NEW YORK ACADEMY OF SCIENCES. UNITED STATES DEC 2002, vol. 979, December 2002 (2002-12), pages 64-75;discussion 76 - 79, XP009027082 ISSN: 0077-8923

### SEQUENCE LISTING

<110> VIB vzw
<120> Transgenic amphibian models for lymphatic vessel development
<130> PCA/Xen/V170
<150> EP 03104123.9
   <151> 2003-11-07
<150> EP 04077583.5
   <151> 2009-09-17
<150> US 60/517,884
   <151> 2003-11-07
<150> US 60/611,705
   <151> 2004-09-22
<160> 9
<170> PatentIn version 3.1
<210> 1
   <211> 23
   <212> DNA
   <213> Artificial Sequence
<220>
   <223> downstream primer
<400> 1
   ggggaaaagt caaatgttct ccg 23
<210> 2
   <211> 20
   <212> DNA
   <213> Artificial Sequence
<220>
   <223> upstream primer
<400> 2
   agttggcgaa tgggcttagc 20
<210> 3
   <211> 31
   <212> DNA
   <213> Artificial Sequence
<220>
   <223> downstream primer
<400> 3
   gttttcatct gaaaagaatg atgcatggat g 31
<210> 4
   <211> 27
   <212> DNA
   <213> Artificial Sequence
<220>
   <223> upstream primer
<400> 4
   acttggaaaa gtcctgggtc gtggagc 27
<210> 5
   <211> 24
   <212> DNA
   <213> Artificial Sequence
<220>
   <223> downstream primer
<400> 5
   gaggcaccac cggatttgac acca 24
<210> 6
   <211> 27
   <212> DNA
   <213> Artificial Sequence
<220>
   <223> upstream primer
<400> 6
   gtggtagtgt tgctggcagg gaacgtc 27
<210> 7
   <211> 25
   <212> DNA
   <213> Artificial Sequence
<220>
   <223> morpholino control oligomer
<400> 7
   cctcttacct cagttacaat ttata 25
<210> 8
   <211> 23
   <212> DNA
   <213> Artificial Sequence
<220>
   <223> x-Prox-1 morpholino oligomer
<400> 8
   cccgtcgagt cgtggtcggg cat 23
<210> 9
   <211> 25
   <212> DNA
   <213> Artificial Sequence
<220>
   <223> Prox-1 antisense morpholino oligonucleotides
<400> 9
   caggcatcac tggactgtta ttgtg 25

## Description

### Field of the invention

The present invention provides molecular tools for the visualization of the lymphatic vessel system in amphibian. Furthermore, the invention provides assays that allow screening for compounds able to modulate lymphangiogenesis.

### Introduction to the invention

Blood vessels are critical for life as they provide oxygen and other vital nutrients to individual cells ¹⁻³. The blood vascular system is a closed pressurized circuit, consisting of arteries, capillaries and veins - transporting the blood to, within and from the tissues, respectively. When blood circulates through the blood vascular system, fluid and proteins unavoidably leak out. A network of lymphatic vessels collects the extravasated bloodless fluid from the tissues and transfers it, as "lymph" back into the venous circulation ⁴⁻¹¹. While 90% of interstitial fluid is absorbed back by blood capillaries, the remaining 10% cannot escape the interstitial space without the functioning of this specialized vascular system of lymphatic vessels. Increased interstitial pressure pulls open initial lymphatic capillaries via their anchoring filaments attached to lymphatic endothelial cells (LECs) and allows the entrance of lymph, which then flows into the collecting lymphatic vessels and is ultimately returned to the blood circulation. At the morphological level, lymphatic microvasculature differs in many respects from the blood vessels. It is characterized by an irregular lumen, a discontinuous or absent basal lamina, overlapping intercellular junctional complexes, the presence of anchoring filaments linking LECs to the extracellular matrix, and relatively sparse and disorganized smooth muscle cell/pericyte investment ⁴⁻⁸. Lymphatic vessels also have an immune function by transporting white blood cells and antigen-presenting cells which patrol the tissues, to the various lymphoid organs, where they elicit immune responses ⁴⁻⁸. Especially the 'superficial' lymphatics in the skin and intestine, which are in contact with foreign antigens, function as transport routes for antigen-presenting Langerhans cells and dendritic cells. The lymphatic vasculature is thus essential for immune defense, as lymph and any foreign material present in it, such as microbial antigens, are filtered through the chain of lymph nodes. In addition to its role in the regulation of fluid balance and immune responses, the intestinal lymphatics called lacteals or chylous vessels, are necessary for the uptake of dietary fat. Unfortunately, malignant cells that escape from their resident tumor, can also traffic along the lymphatic tracts to the lymph nodes and, via entry into the circulation, cause metastatic spread to distant organs.

In view of its important functions, is not surprising that derailed growth or function of the lymphatic system is implicated in numerous diseases, including lymphedema, inflammation/infection, immune diseases, and malignancy ^{4-8,10,14}. Defects of lymphatic development or damage to the lymphatics also lead to disabling and disfiguring swelling of the extremities, as in congenital lymphedema, secondary post-surgical lymphedema or in filariasis. Primary lymphangiogenic and related immune disorders primarily affect children. It is also well recognized that in many cancer types the lymphatic vessels serve as a major route for the metastatic spread of tumour cells - a leading cause of death in patients with cancer, and a major obstacle in the design of effective therapies. As cancer is unfortunately not restricted to the aging, means to prevent lymphatic spread or to minimize radiation- or surgery-induced damage to the lymphatics will require special knowledge, since the biology of disease is often different in children. The aging population of the world is expanding, and they form the majority of those suffering from the many disorders associated with lymphangiogenesis, including cancer, inflammation, infection, and ischemia.

Remarkably, even though lymphatic vessels were already discovered as "milky veins" in 1627 by Gasparo Asellius, yet very limited understanding exists of their development, function and molecular mechanisms underlying their disease processes. The limited progress in lymphangiogenesis research has been partly due to two principal reasons. First, the lack of specific molecular markers has posed insurmountable difficulties in recognizing these vessels in tissues. However, the recent discovery of key lymphangiogenic factors (including VEGF-C and VEGF-D and their receptor VEGFR-3), homeobox transcription factor PROX1, hyaluronan receptor LYVE-1, neuropilin-2 and the membrane protein podoplanin has permitted to obtain novel initial insights into how the lymphatic vessels and blood vessels coordinately grow and affect human disease ^{4-9,11,15,16}. Human studies have also revealed that mutations in the forkhead transcription factor FOXC2 are the cause of lymphedema-distichiasis, a hereditary disease primarily characterized by late-onset lymphedema and a double row of eyelashes ¹⁷⁻¹⁹. In addition, genetic experiments have identified the Tie-2 ligand Angiopoietin-2 and the transcription factor Sox18 as a regulator of lymphatic patterning ^{20,21}.

The second reason why our understanding of the molecular basis of lymphangiogenesis has remained limited is the lack of an appropriate animal model to decipher, on a sufficiently large scale, the function of putative lymphangiogenic gene candidates - a process termed "functional lymphangiogenomics". The knowledge on lymphangiogenesis obtained to date has largely resulted from classical, mostly small-scale studies in *in vitro* models and *in vivo* animal models including lymphangiogenesis models in gene-targeted mice and other experimental animals, or genetic screening of patients. While these models permit to study lymphangiogenesis on a gene-per-gene basis, they are labor-intensive, time-consuming and prohibitively expensive, they do, however, not permit to rapidly and cheaply discover the numerous genes involved in the complex processes of lymphatic vessel formation and function, and their regulation in a living organism. Of all vertebrates, the amphibian *Xenopus* is the lowest vertebrate animal on the evolutionary ladder. Only a limited number of studies have documented the presence of a putative lymphatic vascular network in amphibians but, importantly, solely by using morphological methodologies without any detailed molecular characterization. Conclusive proof of evidence for the lymphatic nature of this vascular network is still lacking. Furthermore, there are conflictive disclosures in the art. Indeed, in tadpoles one article describes the presence of a primitive lymphomyeloid bilateral structure in anuran larvae (Riviere H.B. and Cooper E.L. (Proceedings of the society for Experimental Biology and Medicine (1973) 143 (2) 320-322) while another disclosure refers to the absence of lymph glands in *Rana temporaria* (Plytycz B. and Bigaj J. (1982) Developmental and Comparative Immunology 6(4) 781-784). Furthermore, the mere visualization of lymph glands is by no means a conclusive proof that a functional lymphatic vascular vessel system is present. In the present invention we have developed a reliable method for the efficient visualization of amphibian lymph structures. Surprisingly, in transgenic tadpoles a truly developed lymphatic vascular network can be visualized comprising lymphatic vessels, lymphatic sacs and lymphatic hearts. These transgenic tadpoles are a valuable model for the application of powerful high-throughput genetic tools for the identification and characterization of novel and known genes critically involved in lymphangiogenesis. Furthermore, such a genetic tool can constitute the basis for the development of critically needed new diagnostic and therapeutic approaches for patients with lymphangiogenesis-related diseases.

### Aims and detailed description of the invention

At least four sorts of small animal models have been developed at this moment with proven great potential for functional genomics: the zebrafish *Danio rerio,* the amphibian (frog) *Xenopus,* the fruitfly *Drosophila melanogaster* and the worm *C*. *elegans.* Neither the fruitfly nor the worm has a well-developed vascular system, with sufficient analogy to the mammalian vascular system. Zebrafish are well suited for the study of the blood vascular system but, unfortunately, lack an elaborate lymphatic vascular network and can, therefore not be used to unravel the molecular basis of lymph vessel development. The adult frog, including *Xenopus,* is known to have a lymphatic vascular network but it is important to note that this lymphatic system has only been characterized morphologically ⁴⁷⁻⁵¹. In fact, the lymphatic hearts, e.g. specialized lymphatic sac structures which pump lymph into the venous system, are being used as injection sites to deliver substances to the systemic circulation in adult frogs ^{52,53}. Much less is, however, known about the lymphatic vascular system in the developing tadpole. Only a few preliminary studies have reported the presence of "putative" lymphatic vessels, but the lymphatic nature of these vessels was never conclusively established using appropriate molecular markers ⁵⁴. Conclusive proof of evidence for their lymphatic nature is, therefore, still lacking. Moreover, the functionality of these lymphatic vessels has never been demonstrated. This is critical, as lymphatic vessels in tumors have been reported to be non-functional and it therefore remains unknown whether they are true lymphatic vessels. In the present invention we have developed a molecular tool to visualize the lymphatic vessel system in amphibia, such as *Xenopus.* Said tool can be used for screening compounds involved in lymphangiogenesis. Importantly, *Xenopus* has never been used previously as a genetic model to unravel the molecular basis of lymphangiogenesis. Manipulation of the expression of lymphangiogenic genes, either by over- or underexpression by injecting RNA or morpholino oligomers, or by other transgenic techniques (such as expression of plasmid- or BAC-based transgenes, or viral gene transfer), has also never been previously performed nor achieved. An advantage of using *Xenopus* is the facility to genetically manipulate large numbers of these amphibians at a rapid pace, the embryos are big (allowing easy manipulation), are available in large numbers (thousands per spawning), are accessible at all stages of development, are easy to graft and have a reliable fate map. The multitudes of progeny are easily accessed for rapid and accurate analysis since the eggs are fertilized and develop externally to the mother. Embryos and larvae are therefore available for non-invasive manipulation throughout their development. Another advantage of the *Xenopus* system is that the embryos and tadpoles are essentially transparent. Tools to observe blood, vascular cells and other cellular structures with confocal microscopy at high resolution, or via dynamic imaging with high-resolution time-lapse video microscopic imaging, provide a remarkable resource for genetic and functional studies in *Xenopus.* Microinjection can be used to introduce RNA, DNA or dominant-negative approaches, antisense oligonucleotides, antisense RNAs, short interference RNA (siRNA), antibodies, ribozymes, biologically active molecules, microbead implantation, cell transplantation, fate mapping and cell lineage tracing. More recently, the powerful technique of injecting interfering oligonucleotides (morpholinos) into single-cell embryos allows to efficiently "knock down" one or more genes, thereby elucidating loss-of-function phenotypes *in vivo* in a rapid, inexpensive, and highly efficient manner. Moreover, transgenic embryos can be obtained in a short time and at large scale in *Xenopus tropicalis* and *Xenopus laevis.* In *Xenopus,* transgenes are integrated in the male genome prior to fertilization (unlike in zebrafish and mice) and transgenic embryos can be generated one day and analyzed the next. One can express genes with much better spatial and temporal control in particular tissues, using mouse, human or *Xenopus* promoters. Both model systems can also be used for screening mutagenesis. Simply by adding a mutagen to the tanks or by gene-trapping, literally hundreds of phenotypes may be induced, that can be directly visualized, and selected for further analysis, characterization and identification of the genetic abnormality. Several models of human disease have been identified in this manner. Specific but large-scale phenotypic screens are now available in *Xenopus.* Screening largely relies on the *in situ* staining of marker genes and, thus, any biological process can be studied, as long as the relevant markers are known. In a particular embodiment a gene (e.g. a reporter gene or a biologically relevant transgene) is controlled (expressed) in a stage- and tissue-specific pattern. A preferred method known in the art is the use of the GAL4/UAS system, through the generation of separate UAS-effector and GAL4 'transactivator' transgenic lines (Chae J. et al (2002) Mech. Dev. 117:235 and Grabher C & Wittbrodt J. (2004) BMC Biotechnol. 4(1):26).

For the validation of therapeutic compounds, the transgenic *Xenopus* model of the present invention is also a valuable tool. As models of human disease in *Xenopus* are developed via mutagenesis or transgenic approaches, direct evaluation and high-throughput screening of possible therapeutic agents is feasible. Furthermore, side effects may be quickly assessed prior to pre-clinical testing. Similarly, *in vivo* screens for novel therapeutic molecules may be functionally assessed in these models for human disorders to test for potential therapeutic agents.

Therefore in a first embodiment the invention provides a transgenic amphibian tadpole comprising a gene that is specifically expressed in the lymphatic vessel system of said transgenic amphibian. The word "amphibian" is designated in biology as the grouping of three classes: the *Anura* or *Salienta* (comprising frogs and toads), the *Urodela* or *Caudata* (comprising newts, salamanders and mudpuppies) and the *Gymnophiona* (comprising the caecilians). In a particular embodiment said transgenic amphibian tadpole belongs to the genus *Xenopus. Xenopus* is also better known as the African clawed frog. In a more particular embodiment said *Xenopus* is *Xenopus laevis* or *Xenopus tropicalis.* In yet another particular embodiment said gene that is specifically expressed in the lymphatic vessel system of said transgenic amphibian is a reporter gene. In yet another particular embodiment said gene is a biologically relevant transgene. A biologically relevant transgene is explained hereafter. Expressing biologically relevant transgenes in lymphatic endothelial cells allow to study the role of this biologically relevant transgene in lymphatic vessel development. Similar studies using blood vascular endothelial cell-specific promotors have allowed to unravel the molecular basis of angiogenesis. This technology offers great advantage for deciphering the molecular basis of lymphangiogenesis as well. There are numerous genes which are known to be expressed in lymphatic vessels, but their function remains largely unknown or poorly characterized. This can be accomplished by cloning a construct in which a lymphatic endothelial cell-specific promotor (such as for instance Prox1) drives expression of a lymphangiogenic gene (such as for instance podoplanin). The effects on lymphatic vessel development can then be analyzed by using in situ hybridization to label lymphatic vessels, by using the lymphatic vessel-reporter tadpoles, by performing lymphangiography and by scoring for lymphedema.

In yet another embodiment the invention provides a transgenic *Xenopus* tadpole comprising a reporter gene encoding for a fluorescent protein that is specifically expressed in the lymphatic vessel system of said transgenic Xenopus. The skilled in the art is able to distinguish the developmental stages of Xenopus via the following references: (Nieuwkoop, P.D. & Faber, J. Normal table of Xenopus laevis. Garand Publishing, New York (1994); Sive, H.L., Grainger, R.M. & Harland, R.M. Early Development of Xenopus laevis. A Laboratory Manual, (Cold Spring Harbor Laboratory Press, Cold Spring Harbor, New York, 2000); G Bernardini, M Prati, F Bonetti, G Scari. Atlas of Xenopus development. Springer-Verlag Italia, Milano 1999. ISBN 88-470-0036-X; Peter Hausen and Metta Riebesell. The early development of Xenopus Laevis: An atlas of the histology. Springer-Verlag Berlin, Germany, 1991. ISBN 0-387-53740-6; Tadpoles: the biology of anuran larvae. Edited by Roy W McDiarmid and Ronald Altig. The University of Chicago Press, Chicago and London, 1999. ISBN 0-226-55763-4; Normal table of Xenopus Laevis (Daudin): A systematical and chronological survey of the development from the fertilized egg till the end of metamorphosis. Edited by Pieter D Nieuwkoop and J Faber: with a foreword by John Gerhart and Marc Kirschner. Garland publishing, Inc. New York and London, 1994. ISBN 0-8153-1896-0; Molecular methods in developmental biology: Xenopus and Zebrafish. Edited by Matthew Guille. Humana Press Inc, Totowa, New Jersey, 1999. ISBN 0-89603-790-8 (This book is part of a series called "Methods in molecular biology" and this particular book is vol 127. The editor for the whole series is John M Walker).

The wording a "transgenic *Xenopus"* refers to a *Xenopus* tadpole in which one or more of the cells of said frog contain heterologous polynucleotide sequence (e.g. a reporter gene or a biologically relevant transgene as defined herein) introduced by way of human intervention, such as by transgenic techniques well known in the art. The polynucleotide sequence is introduced into the cell, directly or indirectly, by introduction into a precursor of the cell by way of deliberate genetic manipulation such as microinjection or infection with a recombinant virus. This polynucleotide sequence may be integrated within a chromosome (stable transgenic), or it may be extra-chromosomally replicated (transient transgenic).

A "reporter gene" as used herein refers to a gene that encodes a reporter protein. A reporter protein is any protein that can be specifically detected when expressed. Reporter proteins are useful for detecting or quantitating expression from sequences (Zhang J. et al (2002) Nat. Rev. Mol. Cell. Biol. 3(12): 906) . For example, operatively linking nucleotide sequences encoding a reporter protein to a tissue specific expression sequence allows one to study lineage development. In such studies, the reporter protein serves as a marker for monitoring developmental processes. Many reporter proteins are known to one of skill in the art. These include, but are not limited to β-galactosidase, luciferase, and alkaline phosphatase that produce specific detectable products. Fluorescent reporter proteins can also be used, such as green fluorescent protein (GFP), enhanced green fluorescent protein (eGFP), reef coral fluorescent protein (RCFP), cyan fluorescent protein (CFP), red fluorescent protein (RFP) and yellow fluorescent protein (YFP). For example, by utilizing GFP or RCFP, fluorescence is observed upon exposure to ultraviolet, mercury, xenon, argon or krypton arc light without the addition of a substrate. The use of reporter proteins that, like GFP, are directly detectable without requiring the addition of exogenous factors are preferred for detecting or assessing gene expression during *Xenopus* development. A transgenic *Xenopus* embryo, currying a construct encoding a reporter protein and a lymphatic vessel-specific expression sequence provides a rapid, real time *in vivo* system for analyzing spatial and temporal expression patterns in lymphatic vessels. For example, one skilled in the art would select transgenic *Xenopus* embryos or larvae that express a reporter protein in lymphatic vessels as described in the examples. If the reporter protein is a fluorescent reporter protein, the skilled artisan well see the fluorescent reporter protein expressed in lymphatic vessels.

The wording "specifically expressed in the lymphatic vessel system" means that the reporter gene is expressed predominantly in the lymphatic vessel or lymphatic vascular system, including but not necessarily restricted to lymphatic vessel progenitors, lymphatic vessels, lymphatic sacs and lymphatic hearts. The wording "specifically expressed" does not exclude that there is also some ectopic expression of the reporter gene in for example other organs or structures, as long as the lymphatic vessel system is visualized by said reporter and as long as there is no confusion between the ectopic expression and the lymphatic vessel system by a person skilled in the art. Preferably a reporter gene is expressed predominantly in the lymphatic vessel system when 70%, 80%, 90%, 95% or greater of the observed expression occurs in the lymphatic vessel system. The measuring of said reporter gene expression specifically in the lymphatic vessel system compared to its ectopic expression can be carried out by imaging tools known in the art.

In yet another embodiment the invention provides for a method to produce a transgenic *Xenopus* tadpole comprising introducing a vector comprising a reporter gene under control of a promoter specifically expressed in the lymphatic vascular system into cells of *Xenopus.* Transgenic amphibia that express a reporter protein at specific sites such as the lymphatic vessel system can be produced by introducing (e.g. via DNA injection or retroviral infection) a nucleic acid into fertilized eggs, sperm cells (nuclei), wherein the nucleic acid is under the control of a specific promoter which allows specific expression of the nucleic acid in the lymphatic vessel system. Late-stage embryos can also be produced by nuclear transfer from a variety of donor cells including endoderm, melanophores, keratinised skin cells, neural cells, erythrocytes and intestinal cells. The transgenic amphibia (e.g. *Xenopus*) of this invention can be a transient or a stable transgenic *Xenopus*. The transgenic *Xenopus* in which the expression of a reporter protein is specific for the lymphatic vessel system is contemplated for this invention. The transgenic *Xenopus* utilized in the methods of this invention are produced by introducing a transgenic construct into cells of *Xenopus,* preferably in sperm cells (nuclei) (which are then transplanted into an unfertilized Xenopus egg) or in fertilized eggs. The transgenic construct can be a plasmid or any other sort of vector, including but not restricted to BAC, PAC or YAC, is preferably integrated into the genome of *Xenopus,* however, the construct can also remain episomally (most probably after DNA injection but should not if transgenesis by nuclear transplantation is used). The transgenic construct can be introduced into sperm cells (nuclei) or fertilized eggs or embryonic cells using any technique known in the art. For example, microinjection, electroporation, liposomal delivery, viral delivery and particle gun bombardment can all be utilized to effect transgenic construct delivery to embryonic cells. Embryos can be microinjected at the one or two cell stage or the construct can be incorporated into embryonic cells, which can later be incorporated into a growing embryo. Other methods for achieving *Xenopus* transgenesis that are developed can also be utilized to introduce a construct into an embryo or embryonic cells. In a preferred embodiment introduction of the transgene nucleotide sequence into the *Xenopus* embryo may be accomplished by any means known in the art, but preferably by microinjection (Meng et al. (1999) Methods Cell. Biol. 60, 133-148). Following introduction of the transgene nucleotide sequence into the embryo, the embryo may be incubated for varying periods of time. Incubation to maturity is within the scope of this invention. In addition to similar biological considerations, physical ones also govern the amount (e.g., volume) of exogenous genetic material that can be added to the zygote or to the genetic material. The physical effects of addition must not be so great as to physically destroy the viability of the zygote. The biological limit of the number and variety of DNA sequences will vary depending upon the particular zygote and functions of the exogenous genetic material and will be readily apparent to one skilled in the art, because the genetic material, including the exogenous genetic material, of the resulting zygote must be biologically capable of initiating and maintaining the differentiation and development of the zygote into a functional organism.

The number of copies of the transgene constructs, which are introduced into the zygote, sperm cell (nuclei) or embryonic cells, is dependent upon the total amount of exogenous genetic material added, and will be the amount that enables the genetic transformation to occur. Theoretically only one copy is required, however, generally, numerous copies are utilized, e.g. 1,000-20,000 copies of the transgene construct, in order to insure that one copy is functional. As regards the present invention, there will often be an advantage to having more than one functioning copy of each of the inserted exogenous DNA sequences to enhance the phenotypic expression of the exogenous DNA sequences. Any technique which allows for the addition of the exogenous genetic material into nucleic genetic material can be utilized so long as it is not destructive to the cell, nuclear membrane or other existing cellular or genetic structures. The exogenous genetic material is preferentially inserted into the nucleic genetic material transplantation of genetically modified sperm nuclei or by microinjection. Microinjection of cells and cellular structures is known and used in the art. Progeny of the transgenic animals may be obtained by mating the transgenic animal with a suitable partner, or by *in vitro* fertilization of eggs with sperm obtained from the transgenic animal. Where mating with a partner is to be performed, the partner may or may not be transgenic, where it is transgenic, it may contain the same or a different transgene, or both. Alternatively, the partner may be a parental line. The progeny may be evaluated for the presence of the transgene using methods described above, or other appropriate methods. Retroviral infection can also be used to introduce a transgene into a non-human animal. The developing non-human embryo can be cultured *in vitro* to the blastocyst stage. During this time, the blastomeres can be targets for retroviral infection (Jaenisch (1976) Proc. Natl. Acad. Sci. USA 73, 1260-1264). Alternatively, infection can be performed at a later stage. Virus or virus-producing cells can be injected into the blastocoele (Jahner et al. (1982) Nature 298, 623-628). The viral vector system used to introduce the transgene is typically a replication-defective retrovirus carrying the transgene (Jahner et al. (1985) Proc. Natl. Acad. Sci. USA 82, 6927-6931; Van der Putten et al. (1985) Proc. Natl. Acad. Sci. USA 82, 6148-6152). Following the methods and material described previously, those skilled in the art will be able to retrovirally introduce nucleic acids into *Xenopus* to generate transgenic *Xenopus.* Retroviral gene transfer in Xenopus cell lines and embryos has been done using pantropic and amphotropic vectors: Burns,JC et al. (1996) Retroviral gene transfer in Xenopus cell lines and embryos. In Vitro Cell Dev Biol Anim. Feb;32(2):78-84).

Transgenic *Xenopus* containing a transgene can be identified by numerous methods such as probing the genome of the *Xenopus* for the presence of the transgene construct by Northern or Southern blotting. Polymerase chain reaction and FISH techniques can also be employed to detect the presence of the transgene. Expression of the reporter protein can also be detected by methods known in the art. For example, RNA can be detected using any of numerous nucleic acid detection techniques. Alternatively, an antibody can be used to detect the expression product or one skilled in the art can visualize and quantitate expression of a fluorescent reporter protein such as GFP.

In yet another embodiment the reporter gene is under control of a promoter that is specifically expressed in the lymphatic vascular system. Thus, any promoter or gene sequence which permits expression of the transgene in lymphatic vessels (for example a promoter derived from lymphatic endothelial cells or derived from lymphatic smooth muscle cells) can be used. In a more particular embodiment said promoter is selected from the list comprising Podoplanin promoter, Prox-1 promoter, VEGFR-3 promoter and LYVE-1 promoter. In yet another particular embodiment said promoter is a promoter derived from *Xenopus.* In yet another particular embodiment said promoter is derived from mouse or human. The expression sequences used to drive expression of the reporter proteins can be isolated by one of skill in the art, for example, by screening a genomic *Xenopus* library for sequences upstream of the *Xenopus* gene of interest. The expression sequences can include a promoter, an enhancer, LCR (locus control regions), a silencer and necessary information processing sites, such as ribosome binding sites, RNA splice sites, polyadenylation sites and transcriptional terminator sequences. Alternatively the expression sequences used to drive expression of the reporter proteins can be derived from mice, human or another species known to possess a functional lymphatic vascular network.

In yet another embodiment the invention provides a method for visualizing the lymphatic vessel system in amphibian comprising generating a transgenic amphibian comprising a reporter gene that is specifically expressed in the lymphatic vessel system of said amphibian. In a particular embodiment said transgenic amphibian is *Xenopus laevis* or *Xenopus tropicalis.*

In another embodiment the invention provides the use of a transgenic amphibian, comprising a reporter gene that is specifically expressed in the lymphatic vessel system, to identify a compound capable of modulating lymphatic vessel development comprising (a) contacting said transgenic amphibian with a test compound, (b) comparing the lymphatic vessel system in said transgenic amphibian contacted with said test compound with the lymphatic vessel system of a transgenic amphibian that was not contacted with said test compound and, (c) determining the effect of said test compound on lymphatic vessel development, such that if lymphatic vessel development in the transgenic amphibian contacted with said test compound is different from the lymphatic vessel development in the transgenic amphibian that was not contacted with said test compound, said compound is a modulator of the lymphatic vessel system. In a particular embodiment said transgenic amphibian is a transgenic *Xenopus laevis* or transgenic *Xenopus tropicalis.*

The term "modulating lymphatic vessel development" can also be interchanged by the term "modulating lymphangiogenesis". The word "lymphangiogenesis" is explained in the following. The formation of blood vessels is generally believed to develop via, at least, several mechanisms ^{1,12,13}: (i) "vasculogenesis", referring to the genesis and differentiation of vascular precursors (generally also termed "angioblasts") into endothelial cells which can contribute to vessel growth or enlargement by becoming incorporated into the nascent vessels or by releasing factors modulating their growth or function - this process can occur both in the embryo and adult; (ii) "angiogenesis", referring not only to the sprouting, but also to the remodeling and reshaping of new vessels; and (iii) "arteriogenesis, referring to the maturation and stabilization of vessels via recruitment of mural cells, e.g. pericytes and smooth muscle cells in small and large vessels, respectively; (iv) "collateral vessel growth", referring to the expansive growth of pre-existing collateral vessels. Very likely, lymphatic vessels also grow and remodel in similar ways and mechanisms as blood vessels - and, therefore, could be termed "lymph-vasculogenesis, -angiogenesis, -arteriogenesis and collateral growth" ⁴⁻⁹. It has been recognized that lymphatic vessels arise at later stages in development than blood vessels and that, in fact, particular venous endothelial cells transdifferentiate to lymphatic endothelial cells - a process called "lymphatic commitment" ^{4,5,7-9}. For simplicity, we will use the term "lymphangiogenesis" as a general term to denote, in general, any sort of mechanism whereby lymphatic vessels grow, enlarge or remodel.

By utilizing a transgenic *Xenopus* that expresses a fluorescent reporter protein under the control of a lymphatic vessel specific promoter (*id est* a promoter that is directing the expression specifically in the lymphatic vessel system), the lymphatic vessel system can be visualized in the developing embryo and in later stages of *Xenopus* development. Thus, if a transgenic *Xenopus* of the present invention is exposed to a compound (e.g. a small compound or a morpholino) the effect on the lymphatic vessel system should be readily apparent by monitoring the fluorescence. Transgenic *Xenopus* embryos can be "cultured" in 96 well plates where they can for example be soaked in test compounds. The effects of the test compound can be readily visualized on the effect on lymphatic vessel development.

In order to assess the modulation properties of a particular test compound on lymphatic vessel development, one would contact the transgenic *Xenopus* with said test compound. The effects of the test compound are assessed by observing detectable spatial and temporal changes in fluorescence between a contacted and non-contacted transgenic *Xenopus* with the particular test compound. For example, an increase in fluorescence at a particular site is observed after addition of a test compound, i.e. increased fluorescence as a result of an enhanced lymphatic vessel system. Other changes that one of skill in the art would observe include a decrease fluorescence in a particular lymphatic vessel system because of a disappearance of a particular lymphatic vessel system. Yet another change is the monitoring of a particular change in lymphatic vessel appearance (e.g. lymph structures disappear or new structures are formed). Upon identification of a gene involved in lymphatic vessel function, the present invention also contemplates knocking out or knocking down said genes in *Xenopus* in order to determine their role in lymphangiogenesis. As used herein, a "knock-out" can be a gene knockdown or the gene can be knocked out by a mutation such as, a point mutation, an insertion, a deletion, a frameshift, or a missense mutation by techniques known in the art. Knockouts can also be effected by utilizing morpholino technology.

As utilized herein, a "compound" can be but is not limited to a chemical, a small molecule, a drug, an antibody, a peptide, a secreted protein, a nucleic acid (such as DNA, RNA, a polynucleotide, an oligonucleotide or a cDNA) or an antisense RNA molecule, a ribozyme, an RNA interference nucleotide sequence, an antisense oligomer or a morpholino.

The following section clarifies the use of compounds.

Temporally and tissue-specific gene inactivation has also been achieved through the creation of transgenic organisms expressing antisense RNA. The successful use of antisense transgenics has been reported for several cases in plants, rats and mice. An antisense construct can be delivered, for example, as an expression plasmid, which, when transcribed in the cell, produces RNA that is complementary to at least a unique portion of the cellular mRNA, which encodes a polypeptide, suspected to have a role in lymphangiogenesis. A more rapid method for the inhibition of gene expression is based on the use of shorter antisense oligomers consisting of DNA, or other synthetic structural types such as phosphorothiates, 2'-0-alkylribonucleotide chimeras, locked nucleic acid (LNA), peptide nucleic acid (PNA), or morpholinos. With the exception of RNA oligomers, PNAs and morpholinos, all other antisense oligomers act in eukaryotic cells through the mechanism of RNase H-mediated target cleavage. PNAs and morpholinos bind complementary DNA and RNA targets with high affinity and specificity, and thus act through a simple steric blockade of the RNA translational machinery, and appear to be completely resistant to nuclease attack. An "antisense oligomer" refers to an antisense molecule or anti-gene agent that comprises an oligomer of at least about 10 nucleotides in length. In embodiments an antisense oligomer comprises at least 15, 18 20, 25, 30, 35, 40, or 50 nucleotides. Antisense approaches involve the design of oligonucleotides (either DNA or RNA, or derivatives thereof) that are complementary to an mRNA encoded by polynucleotide sequences selected with a putative role in lymphangiogenesis. The antisense oligomers will bind to said mRNA transcripts and prevent translation. Absolute complementarity, although preferred, is not required. A sequence "complementary" to a portion of an RNA, as referred to herein, means a sequence having sufficient complementarity to be able to hybridize with the RNA, forming a stable duplex; in the case of double-stranded antisense polynucleotide sequences, a single strand of the duplex DNA may thus be tested, or triplex formation may be assayed. The ability to hybridize will depend on both the degree of complementarity and the length of the antisense polynucleotide sequence. Generally, the longer the hybridizing polynucleotide sequence, the more base mismatches with an RNA it may contain and still form a stable duplex (or triplex, as the case may be). One skilled in the art can ascertain a tolerable degree of mismatch by use of standard procedures to determine the melting point of the hybridized complex. Oligomers that are complementary to the 5' end of the message, e.g., the 5' untranslated region (UTR) up to and including the AUG translation initiation codon, should work most efficiently at inhibiting translation. However, sequences complementary to the 3' UTR of mRNAs have recently been shown to be effective at inhibiting translation of mRNAs as well (Wagner, R. (1994) Nature 372, 333-335). Therefore, oligomers complementary to either the 5', 3' UTRs, or non-coding regions of a said novel gene could be used in an antisense approach to inhibit translation of said endogenous mRNA encoded by polynucleotides with a putative role in lymphangiogenesis. Oligomers complementary to the 5' UTR of said mRNA should include the complement of the AUG start codon. Antisense oligomers complementary to mRNA coding regions are less efficient inhibitors of translation but could be used in accordance with the invention. Whether designed to hybridize to the 5', 3' or non-coding region of a said mRNA, antisense oligomers should be at least 10 nucleotides in length, and are preferably oligomers ranging from 15 to about 50 nucleotides in length. In certain embodiments, the oligomer is at least 15 nucleotides, at least 18 nucleotides, at least 20 nucleotides, at least 25 nucleotides, at least 30 nucleotides, at least 35 nucleotides, at least 40 nucleotides, or at least 50 nucleotides in length. A related method uses ribozymes instead of antisense RNA. Ribozymes are catalytic RNA molecules with enzyme-like cleavage properties that can be designed to target specific RNA sequences. Successful target gene inactivation, including temporally and tissue-specific gene inactivation, using ribozymes has been reported in mouse, zebrafish and fruitflies. RNA interference (RNAi) is a form of post-transcriptional gene silencing. The phenomenon of RNA interference was first observed and described in *Caenorhabditis elegans* where exogenous double-stranded RNA (dsRNA) was shown to specifically and potently disrupt the activity of genes containing homologous sequences through a mechanism that induces rapid degradation of the target RNA. Several reports describe the same phenomenon in other organisms, including experiments demonstrating spatial and/or temporal control of gene inactivation, including plant (*Arabidopsis thaliana*), protozoan (*Trypanosoma bruceii*), invertebrate (*Drosophila melanogaster*), and vertebrate species (*Danio rerio* and *Xenopus laevis*). The mediators of sequence-specific messenger RNA degradation are small interfering RNAs (siRNAs) generated by ribonuclease III cleavage from longer dsRNAs. Generally, the length of siRNAs is between 20-25 nucleotides (Elbashir et al. (2001) Nature 411, 494-498). More recently it has been shown that morpholino antisense oligonucleotides in zebrafish and frogs overcome the limitations of RNase H-competent antisense oligonucleotides, which include numerous non-specific effects due to the non target-specific cleavage of other mRNA molecules caused by the low stringency requirements of RNase H. Morpholino oligomers therefore represent an important new class of antisense molecule. Oligomers of the invention may be synthesized by standard methods known in the art. As examples, phosphorothioate oligomers may be synthesized by the method of Stein et al. (1988) Nucleic Acids Res. 16, 3209-3021), methylphosphonate oligomers can be prepared by use of controlled pore glass polymer supports (Sarin et al. (1988) Proc. Natl. Acad. Sci. USA. 85, 7448-7451). Morpholino oligomers may be synthesized by the method of Summerton and Weller U.S. Patent Nos. 5,217,866 and 5,185,444.

In a particular embodiment the transgenic *Xenopus* comprising a reporter gene that is specifically expressed in the lymphatic vessel system of said *Xenopus* can be used to identify genes that play a role in lymphatic dysfunction. In another particular embodiment said transgenic *Xenopus* can be used to identify genes that play a role in defects of the lymph vasculature. In yet another particular embodiment said transgenic *Xenopus* can be used to identify genes that play a role in diseases related to a defect in lymph vessels.

The wording 'lymph vessels', 'lymphatic vessels', 'lymphatic vessel structure', 'lymphatic structure' can be used interchangeable in the present invention.

To further clarify the present invention some terms are clarified below:

"A defect in lymph vasculature" relates to an alteration in normal lymph vessel formation within any given animal. In the context of the invention, this includes but is not limited to, a lack or significant decrease of lymph vessel formation, an overabundance of lymph vessels, invasive lymph vessel formation (amount of vessels formed is normal, however formation follows no defined pattern), abnormally slow or rapid lymphangiogenesis, arrested lymphangiogenesis, or abnormal lymph vessel wall formation (e.g. structural defects such as leakiness).

"A disease related to a defect in lymph vessels" encompasses any disease, ailment, or malady that is the result of and/or maintained by defects during lymphatic development (lymphangiogenesis), remodeling of lymph vessel formation, or structural defects of lymph vessels (e.g. tumor growth).

The term 'pathological lymphangiogenesis' refers to the formation and growth of lymph vessels during the maintenance and the progression of several disease states. One important disease state is cancer and more particularly metastasis. More specific conditions where pathological lymphangiogenesis can occur are in lymph vessels (atherosclerosis, hemangioma, hemangioendothelioma), bone and joints (rheumatoid arthritis, synovitis, bone and cartilage destruction, osteomyelitis, pannus growth, osteophyte formation, neoplasms and metastasis), skin (warts, pyogenic granulomas, hair growth, Kaposi's sarcoma, scar keloids, allergic oedema, neoplasms), liver, kidney, lung, ear and other epithelia (inflammatory and infectious processes (including hepatitis, glomerulonephritis, pneumonia), asthma, nasal polyps, otitis, transplantation, liver regeneration, neoplasms and metastasis), uterus, ovary and placenta (dysfunctional uterine bleeding (due to intrauterine contraceptive devices), follicular cyst formation, ovarian hyperstimulation syndrome, endometriosis, neoplasms), brain, nerves and eye (retinopathy of prematurity, diabetic retinopathy, choroidal and other intraocular disorders, leukomalacia, neoplasms and metastasis), heart and skeletal muscle due to work overload, adipose tissue (obesity), endocrine organs (thyroiditis, thyroid enlargement, pancreas transplantation), hematopoiesis (AIDS (Kaposi), hematologic malignancies (leukemias, etc.), lymph vessels (tumour metastasis, lymphoproliferative disorders).

"Lymphatic dysfunction" comprises disorders due to an underlying lymphatic failure. Lymphatic failures generally result in lymphatic edema (lymphedema). Lymphedema is an external manifestation of 'lymphatic failure' (lymphatic dysfunction). This failure or dysfunction can be viewed as caused by an underlying 'abnormal growth' (dysplasia) of the lymphatic vessels, either a primary aplasia, hypoplasia or hyperplasia, or a secondary (acquired) obstructive, obliterative, or dysfunctional process with inadequate compensatory lymphatic growth and collateral formation to handle the normal, reduced, or increased volume of lymph. Other examples of aberrant (dysfunctional) lymphangiogenesis comprise congenital hereditary lymphedema which occurs sporadically in dogs and several other species, lymphedema-angiodysplasia (LE)AD) syndromes which span a wide spectrum of poorly understood lymphatic developmental and growth disorders that range from familial, chromosomal, and sporadic origin to acquired forms, lymphangiodysplasia, lymphangiomas, lymphangioleiomyomatosis.

The present invention is more particularly described in the following examples, which are intended as illustrative only since numerous modifications and variations therein will be apparent to those skilled in the art.

### Examples

### 1. Molecular characterization of a lymphatic vascular system in the tadpole

In the present invention we have established for the first time the presence of lymphatic vessels, by using molecular *in situ* hybridization of tadpoles at various embryonic stages with molecular probes, known to mark lymphatic vessels in the mouse. Therefore, the *Xenopus* orthologue sequences of murine Prox-1 (termed from hereon as "x-Prox-1") and VEGF receptor-3 (termed "x-VEGFR-3") were cloned. Hybridization was performed using: (i) a 950 nucleotide (nct) fragment of the x-Prox-1 cDNA (nucleotide 190 till 1139 relative to the ATG start codon), obtained by RT-PCR amplification of tadpole RNA using the following primers (downstream primer: 5'GGG GAA AAG TCA AAT GTT CTC CG 3'; upstream primer: 5' AGT TGG CGA ATG GGC TTA GC 3', derived from NCBI# AB008773 gene sequence); (ii) a 1552 bps fragment of the x-VEGFR-3 cDNA, obtained by RT-PCR amplification of tadpole RNA using the following primers (downstream primer: 5' GTT TTC ATC TGA AAA GAA TGA TGC ATG GAT G 3' ; upstream primer: 5' ACT TGG AAA AGT CCT GGG TCG TGG AGC 3', derived from the genbank EST # BM261245). Whole mount *in situ* hybridization with digoxigenin-labeled antisense RNA probes was carried out according to previously reported protocols ^{55,56}. Briefly, antisense RNA probes were synthesized using digoxigenin labeling mix (Boehringer Mannheim). Unincorporated nucleotides were removed by gel filtration through G-50 Sephadex columns (Mini Quick Spin Columns, Boehringer Mannheim). Embryos from different developmental stages were collected and fixed for 2 h in MEMFA fix and stored in 100% ethanol -20 C until further use. Following rehydration, the embryos were treated with proteinase K (10 µg/ml) to increase sensitivity and triethanolamine/acetic anhydride to inhibit endogenous phosphatases. Hybridizations with digoxigenin- labeled antisense RNA probes (0.5µg/ml) were carried out at 60°C overnight. Sheep anti-digoxigenin Fab fragments conjugated to alkaline phosphatase (Boehringer Mannheim) were used to detect the digoxigenin-labeled probes. Color development was performed using BM purple (Boehringer Mannheim). Embryos were bleached to diminish pigmentation and cleared in 100% ethanol before photographic documentation. After staining, tadpoles were cross-sectioned and counterstained. In addition, we also used an anti-murine Prox-1 antibody (which crossreacts with x-Prox-1) to immunolabel lymphatic vessels on cross-sections. This analysis revealed the presence of Prox-1 positive vascular structures both rostrally and caudally in the stage 35-36 tadpole. In addition, the anlage of a lymphatic heart could be clearly visualized in the jugular region, dorsally of the promesonephric tubules. As in the mouse, expression of Prox-1 was found to be first confined to the external region of the cardinal vein, indicating lymphatic commitment of venous endothelial cells. At later stages (e.g. stage 40), the ventral and dorsal caudal lymphatic vessels in the tail of the tadpole also stained positive, when *in situ* hybridized using the x-Prox-1 or x-VEGFR-3 probe. The ventral caudal lymphatic vessel lied ventrally of the posterior cardinal vein - their close proximity further suggesting that the lymphatic vessel derived from the vein.

### 2. Genetic manipulation of the lymphatic vascular system in the tadpole

In the present invention we have provided unprecedented experimental proof of evidence that the tadpole can be genetically manipulated to study the molecular basis of lymphangiogenesis and evaluate the function of lymphangiogenic genes. This was achieved in the following way:

The first method involves the overexpression of x-VEGF-C, a well-known key molecule regulating lymphangiogenesis in other animals. Overexpression of VEGF-C is known to stimulate lymphangiogenesis in health and disease. We employed tadpole RNA to amplify by RT-PCR (using the downstream primer 5' GAG GCA CCA CCG GAT TTG ACA CCA 3' and upstream primer 5' GTG GTA GTG TTG CTG GCA GGG AAC GTC 3') the open reading frame, encoding the mature form of x-VEGF-C (sequence information public available as EST NCBI# CA973641). This sequence, preceded by an ATG initiation codon, a Kozak and signal peptide sequence was cloned into the pCS2⁺ expression vector, containing the ubiquitously and constitutively active CMV promoter. *Xenopus* eggs were obtained and fertilized by standard methods ⁵⁷, de-jellied with 2% cysteine (pH 8.0), rinsed, and placed in 1 xMMR + 6% Ficoll for injections. In vitro transcribed x-VEGF-C RNA (1.0 ng in 5 nl volume) was then injected into the ventral marginal blastomeres of either 8-cell stage or 16-cell stage *Xenopus* embryos to target the expression of VEGF-C to the putative jugular region of developing *Xenopus* embryos. Embryos were staged according to the table of *Xenopus laevis* development ⁵⁸. Labeling of the lymphatic endothelial cells by *in situ* Prox-1 hybridization of transgenic tadpoles at stage 33-34 revealed that the lymphatic sac in the jugular region was dilated and hyperplastic, with more numerous Prox-1 positive lymphatic endothelial cells than in embryos after control DNA injection.

In a second example 25 ng of Prox-1 antisense morpholino oligonucleotides (Prox-1 MOs) was microinjected in a 1-cell stage *Xenopus,* in order to investigate the influence on the development of the lymphatic system. Prox1 is known to be expressed exclusively in lymphatic but not in blood vascular endothelial cells. *Xenopus laevis* embryos were obtained by artificial fertilization using standard techniques and sexually mature adult frogs purchased from Nasco Biology (Fort Atkinson, WI). The Prox-1 MOs, 5'-CAGGCATCACTGGACTGTTATTGTG-3', were purchased from Gene Tools (Corvallis, OR) and designed around the ATG codon. As ATG-specific morpholinos lower translation - but not transcription - of the target gene, we used whole embryo mount *in situ* hybridisation for Prox1 to visualize the defects in lymphatic vascular development. The 5' nucleotide sequence of Prox-1 was obtained using 5' RACE using primers based on published *Xenopus laevis* cDNA (GenBank # AB008773). To visualise Prox-1 expression pattern, embryos were collected at different developmental stages (according to Nieuwkoop and Faber), fixed and submitted to whole-mount *in situ* hybridisation according Harland (Harland, 1991), with the exception that BM purple was used as a substrate. The Prox-1 digoxigenin-labeled riboprobe used was constructed from a 950 bp cDNA fragment (GenBank # AB008773), which was cloned into pGEMTeasy and linearised with Aatll to get SP6 antisense probe. After in situ hybridisation the embryos where bleached, cleared and stored in glycerol until analysed. Analysis was performed using a Zeiss Axioplan 2 imaging microscope equipped with a Axiocam HrC camera and the KS300 morphometry software. The total ISH-region was divided into 3 different regions where the first region represents the ventral side the tail, a second region where the dorsal migration of prox-1 positive cells takes place and finally a third region which represents the dorsal side. For all regions, length of positive staining was measured, followed by segmentation of the ISH-area by hue, lightness and saturation for defining the true stained area. A value of migration is given by the cell that travelled furthest across the tail. In non-morpholino treated *Xenopus* we observed that the normal lymphatic system starts developing at the jugular region of the tadpole around developmental stage 29/30. Apart from expression in non-lymphatic tissues such as the pronephros and the eye, the Prox-1 expression at this stage seems to be restricted to the jugular region. At the following stage (31-32), the staining in the jugular region is expanded to what will become the future lymphsacs. The region of the future anterior lymph hearts as well as the ventral caudal region show intense Prox-1 positive signal too. By stage 33/34 the prox positive cells in the caudal region start the formation of the ventral caudal lymphatic vessel. Prox-1 positive lymphatic cells are migrating from the ventral to the dorsal region and this event is culminating at stage 35/36 where the highest amount of migrating cells can be accounted for. The formation of what eventually will become the dorsal lymphatic vessel is also initiated at this stage. By stage 37-38 a majority of the prox positive cells have reached the dorsal side where they are contributing to the formation of dorsal caudal lymphatic vessels. In Prox-1 morpholino treated *Xenopus* (designated as Prox-1 morphants or Prox-1 knockdowns) although we observed that Prox-1 staining of the anterior region is clearly reduced we have in particular focused on the analysis of the tail region of the tadpoles. In the tail of the Prox-1 morphants dorsal migration of Pro-1 positive cells is clearly impaired. This is most apparent for stage 35/36 where migration is significantly reduced with about 55%. Though to a lesser degree, the reduction is also significantly reduced for stage 37/38. The total Prox-1 positive area is also significantly reduced in about 60% of the Prox-1 morphants at stage 33/34, 50% for stage 35/36 and 35% for stage 37/38 as compared to control tadpoles. This is in agreement with the phenotype Prox-1 null mice where the development of the lymphatic vasculature has been found to be affected due to an arrest of the budding and migration of the prox positive structures (Wigle JT and Oliver G (1999) Cell, 98(6):769-78. Knockdown of Prox1 also impaired the formation of the lymph hearts and the rostral lymphatic structures. Interestingly, manifest edema in the head and anterior trunk developed in most Prox1 knockdown embryos beyond stage 45, when the lymphatic circulation is functional in control tadpoles. Upon macroscopic inspection or microscopic analysis, we could not detect any signs of extravasation of red blood cells, further indicating that the swelling represents lymphedema, resulting from insufficient lymph drainage. The lymphatic defects were not attributable to defects in cardiogenesis (looping, septation and beating of the heart, and blood circulation were all normal in Prox1 morfants, neither were they secondary to abnormal angiogenesis. Indeed, when scoring angiogenesis by counting the number of Msr-positive intersomitic vessels (ISVs) in the anterior trunk and measuring the Msr-positive area in the posterior trunk, development of blood vessels was not affected in Prox1 morphants. Msr is the mesenchyme-associated serpentine receptor, which is a homologue of the G protein-coupled receptor angiontensin-receptor related protein, and is specifically expressed by blood vascular endothelial cells.

### 3. Specificity of lymphatic vessel identity

To confirm that lymphatic vessels constitute indeed a separate network, distinct from blood vessels, we tested a large set of available antibodies, labeling blood and lymphatic endothelial cells in chicken, mice and humans, for possible cross-reactivity in Xenopus tadpoles. Of all antibodies tested, an anti-human von Willebrand factor (vWF) was found to stain lymphatic vessels in tadpoles. This antibody is known to label blood vascular endothelial cells and, albeit weaker, also lymphatic endothelial cells in murine tissues. Whole mount immunostaining of tadpoles revealed that the vWF antibody strongly labeled the large blood vessels but also, though less strongly, lymphatic vessels. For instance, in the posterior trunk, the PCV (paired posterior cardinal veins) was strongly vWF-positive, while the adjacent VCLV (ventral caudal lymphatic vessel) was vWF-immunoreactive, though weaker. On cross-sections, only the VCLV, but not the PCV, was immunoreactive for Prox1. Another characteristic of lymphatic vessels is that, unlike blood vessels, they lack a well-established basement membrane. Staining of transverse sections revealed that only the PCV, but not the Prox1-positive VCLV, was immunoreactive for laminin, a basement membrane component. In addition, analysis of semi-thin sections revealed that endothelial cells in the PCV were closely juxtaposed and formed tight connections, while they were only loosely assembled in the VCLV, with intercellular gaps. Furthermore, red blood cells were only detected in blood vessels but never in lymphatic vessels.

### 4. Quantification of lymphangiogenesis in tadpoles

We next explored whether the Xenopus tadpole model would allow us to explore the function of lymphangiogenic gene candidates by knocking down their expression after injection of morpholino oligomers into fertilized eggs. For this model to be useful, lymphatic vessel defects should be reproducibly quantified. We therefore measured morphometrically the area of Prox1-positive cells in the caudal trunk and rostral head at various stages of embryonic development. The area of Prox1-positive cells in the rostral head region at stage 32 and in the ventral posterior trunk at stage 33/34 is a measure of lymphatic commitment via transdifferentiation of venous into lymphatic endothelial (progenitor) cells (see above). The area of Prox1-positive lymphatic cells, migrating from the PCV to the dorsal roof in the posterior trunk at stage 35/36 is a parameter of lymphatic cell migration, while the area of Prox1-positive cells at the dorsal roof branching out into the DCLV (Dorsal Caudal Lymphatic Vessel) at stage 37/38 is a measure of lymphatic sprouting/branching. We also measured the maximal distance, that Prox1-positive cells migrated from the PCV to their far most dorsal endpoint. Validation of this novel scoring method in control tadpoles revealed highly reproducible measurements within and between experiments, when analyzing stage-matched tadpoles. In the posterior trunk, the area where lymphatic endothelial cells arise was the first to develop at stage 33/34 and doubled in size by stage 37/38. The area of migrating lymphatic endothelial cells in this region was minimal at stage 33/34 and increased 5-fold by stage 37/38, while the area of the sprouting lymphatic vessels was substantial by stage 37/38.

### 5. The lymphatic vasculature is functional

We next determined whether the Prox1-positive vessels were functional lymphatic vessels, capable of draining extravasated fluid. In contrast to blood vessels, lymphatic vessels are well known to selectively absorb dyes from the surrounding interstitium and can thus be labeled upon injection of dyes. We therefore injected subcutaneously FITC-dextran at various sites in the posterior trunk of stage 46-54 tadpoles and examined, by time-lapse videomicroscopy, the spatial distribution of the green fluoroscent dye. In stage 46 tadpoles, both the DCLV (dorsal caudal lymphatic vessel) and VCLV (ventral caudal lymphatic vessel) were capable of taking up FITC-dextran and draining progressively the dye rostrally towards the beating lymph heart. In general, only the DCLV absorbed the dye when injected topically in the dorsal trunk, while only the VCLV drained the dye upon topical injection in the ventral trunk, thus indicating that the dye is locally absorbed. In older tadpoles (stage 54), small side branches of the DCLV and

VCLV, branching off in the dorsal and ventral fin respectively, were also filled with FITC-dextran, suggesting that these lymphatic vessels lack functional valves. By stage 56, tadpoles also developed paired caudal lateral lymphatic vessels (CLLV), which contained numerous side-branches, which likely formed by sprouting. These CLLVs were only visible when FITC-dextran was injected in the anterior but not yet in the posterior part of the tadpoles. According to Hoyer, these lateral caudal lymphatics arise from a superficial reticular lymphatic vascular network in the proximal trunk region, lateral to the myomers, and end blindly, forming reticular branches. As in avian species, the lymphatics of the body wall in tadpoles seem to develop independently from the deep VCLV and DCLV lymphatics by *in situ* differentiation of scattered Prox1-positive mesodermal cells in the dermatome. Compared to blood flow, lymph flow in the CLLVs was much slower (50 µm/minute). To unambiguously demonstrate that the lymphatic vessels, labeled by FITC-dextran, were not blood vessels, we combined angio- and lymphangiography in a single tadpole to visualize both perfused blood and lymphatic vessels. Angiography was accomplished by injecting, intracardially, a red fluorescent dye (tetramethylrhodamine-dextran) in stage 54 embryos. Large and small blood vessels were visible upon angiography. In the trunk, the red dye labeled the dorsal aorta, PCV and dorsal longitudinal anastomosing vessel (DLAV), as well as the intersomitic vessels and side-branches in the fins. Angiography, followed by lymphangiography in the same tadpole, clearly revealed that blood vessels and lymphatic vessels were distinct non-overlapping structures. For instance, in the ventral trunk, the PCV formed dorsally of the VCLV, consistent with the histological findings. While the above experiments showed that a protein, exogenously administered to the interstitium, is absorbed by lymphatic vessels, they did not establish whether a blood-borne protein would be also reabsorbed by lymphatic vessels after leaking out of blood vessels. We therefore injected intracardially in stage 45 embryos tetramethylrhodamine-isolectin, which extravasates from blood vessels, and monitored by time-lapse videomicroscopy its spatial distribution. Within seconds after intracardial injection, blood vessels such as the PCV in the trunk were labeled. However, after 10 minutes, traces of the red dye were clearly detectable in the VCLV, showing indeed that the extravasated isolectin was reabsorbed by lymphatic vessels. Unlike mammals, lymph hearts in adult frogs pump the lymph from the lymphatic into the venous vascular circulation, but very little is known whether these structures already function in tadpoles. Upon injection of FITC-dextran, the green fluorescent dye was indeed ejected by the lymph hearts in jet-like streams into the pronephric sinuses, thus showing that the lymphatic network is functional by stage 42. Notably, FITC-dextran injected in the trunk never reached the rostral head region and, conversely, when injected into the head region, the dye was never detected in the trunk, thus suggesting that a valve in the lymph heart prevented backflow. Indeed, lymphangio-video microscopy suggested the movement of a valve-like structure in the lymph heart at the junction between the lymphatic and venous circulation.

### 6. Generation of transgenic tadpoles, expressing a fluorescent gene product in their lymphatic vessels

To visualize the lymphatic vascular network, several strategies have been used to express a marker gene in lymphatic endothelial cells.

The first method involves the construction of transgenes, whereby expression of the eGFP cDNA open reading frame is driven by a promoter fragment of a gene, which is selectively or restrictedly expressed in lymphatic endothelial cells. The following promoter fragments have been used (nucleotides are numbered relative to the ATG start codon as "1"):
(i) a fragment encompassing nct -1654 to -1 and another containing nct -3417 to +3 of the murine podoplanin promoter were amplified by PCR using a public available BAC as template (Ensemble reference #: ENSMUSG00000028583 / AL611982; BAC#RP23-348F1); (ii) a fragment encompassing nct -573 to +39 and another containing nct -2538 to +39 of the murine Prox-1 promoter was amplified by PCR using a public available BAC as template (Ensemble reference #: ENSMUSG00000010175 / AC026911; BAC#RP23-190F21); (iii) a preferred fragment is a fragment encompassing nct -9970 to -8027 of the murine prox1 promotor which was amplified by PCR using a public available BAC as template (Ensemble reference #: ENSMUSG00000010175 / AC026911; BAC#RP23-190F21); (iv) a fragment encompassing nct -899 to +3 of the murine VEGFR-3 promoter was amplified by PCR using a public available BAC as template (Ensemble reference #: ENSMUSG00000020357 /AL646088; BAC#RP23-58E13); (v) a fragment encompassing nct -1531 to +3 of the murine LYVE-1 promoter was amplified by PCR using a public available BAC as template (Ensemble reference#: ENSMUSG00000030787 / AC100695; BAC#RP23-168N18). These PCR fragments were subsequently cloned as SalI/BamHI or SalI/BglII fragments in front of a BamHI/EcoRI fragment encoding GFP (amplified from the pShuttle hrGFP-2 plasmid; Stratagene) into the pCS2⁺ expression vector after prior removal of the SalI/BamHI CMV promoter fragment. Hundred pg of these plasmids was injected in the ventral marginal blastomeres of 16-cell stage embryos to target expression of the GFP signal to the putative jugular region of developing Xenopus tadpole. In addition, these plasmids have been used in transgenesis procedure by transplantation of a genetically manipulated sperm nucleus into an unfertilized Xenopus egg to generate transgenic tadpoles. Transgenic animals were prepared by the method of Kroll and Amaya ⁶⁰. Briefly, one testis was dissected from an anesthetized male frog, rinsed in 1xMMR, macerated in NPB buffer and filtered through the 40 µm Nylon cell strainer (Becton Dictinson). The sperm suspension was centrifuged at 3000 rpm for 15 min at 4°C in a Sorvall HB-4 rotor, pelleted sperm was resuspended in 1 ml of NPB and permeabilized with 50 µg/ml of digitonin for 5 min at room temperature. Sperm nuclei were washed in ice-cold NPB/3%BSA buffer, centrifuged at 3000 rpm for 15 min at 4°C and resuspended in SDB buffer. Demembraned sperm nuclei were incubated at room temperature first for 5 min with 250 ng of Not I-linearized plasmid DNA followed by 15 min incubation with the high speed egg extract ⁶¹ which partially decondenses the sperm chromatin and in the presence of 0.5 unit of Not I restriction enzyme to facilitate integration by causing breaks in the sperm chromatin. The manipulated sperm nuclei were transplanted into unfertilized eggs with the large tip glass capillary needle (80 µm diameter) connected to the infusion syringe pomp (Harvard Apparatus). After two hours normally cleaving embryos were gently separated from uncleaved eggs and transfer to the 0.1xMMR + 6% Ficoll + 50 µg/ml gentamycin and grown overnight at 18 °C. Normally developing transgenic embryos were further grown in 0.1x MMR+ 50 µg/ml gentamycin and monitor for the GFP signal under the fluorescence stereomicroscope (LeicaMZFLIII).

The second method involves the construction of BAC transgenes, whereby expression of the eGFP cDNA open reading frame is driven by the entire endogenous promoter of a lymphatic-selective gene in a 100-200 kb BAC. The advantage of using BACs is that the presence of the entire endogenous promoter and other gene-expression regulatory regions in these BACs permit to obtain spatio-temporal regulation of expression of the transgene, similar as that of the endogenous gene. Therefore, a BACs containing the murine Prox-1 gene (245 kb; Ensemble reference #: ENSMUSG00000010175 / AC026911 BAC#RP23-190F21) has been engineered, using previously established recombineering methods, such that the entire coding region of GFP is positioned at nct -59 (relative to the ATG start codon) and driven by the endogenous Prox-1 promoter. To inactivate the murine Prox-1 gene in the BAC, part of exon 1 of murine Prox-1 has been deleted. Similarly, a BAC containing the human LYVE-1 gene (156 kb; Ensemble reference #: ENSG00000133800 / AC021914 BAC#RP11-68C8) has been engineered such that the entire coding region of GFP is positioned at nct -1 (relative to the ATG start codon) and driven by the endogenous LYVE-1 promoter; the entire first exon and part of the downstream intron of the endogenous human LYVE-1 gene have been deleted. These DNA constructs are then used for transgenesis, similar as described for the transgene plasmids. High-resolution confocal imaging, combined with three-dimensional reconstruction, of transgenic Prox1-GFP tadpoles reveals green fluorescence in locations of Prox1 expression (as analyzed by *in situ* hybridization).

### References

1. Carmeliet, P. Angiogenesis in health and disease. Nat Med 9, 653-60 (2003).
2. Kerbel, R. & Folkman, J. Clinical translation of angiogenesis inhibitors. Nat Rev Cancer 2, 727-39 (2002).
3. Carmeliet, P. & Jain, R.K. Angiogenesis in cancer and other diseases. Nature 407, 249-57 (2000).
4. Alitalo, K. & Carmeliet, P. Molecular mechanisms of lymphangiogenesis in health and disease. Cancer Cell 1, 219-27 (2002).
5. Stacker, S.A., Achen, M.G., Jussila, L., Baldwin, M.E. & Alitalo, K. Lymphangiogenesis and cancer metastasis. Nat Rev Cancer 2, 573-83 (2002).
6. Karkkainen, M.J., Makinen, T. & Alitalo, K. Lymphatic endothelium: a new frontier of metastasis research. Nat Cell Biol 4, E2-5 (2002).
7. Oliver, G. & Detmar, M. The rediscovery of the lymphatic system: old and new insights into the development and biological function of the lymphatic vasculature. Genes Dev 16, 773-83 (2002).
8. Streit, M. & Detmar, M. Angiogenesis, lymphangiogenesis, and melanoma metastasis. Oncogene 22, 3172-9 (2003).
9. Wilting, J., Christ, B., Yuan, L. & Eichmann, A. Cellular and molecular mechanisms of embryonic haemangiogenesis and lymphangiogenesis. Naturwissenschaften 90, 433-48 (2003).
10. Cassella, M. & Skobe, M. Lymphatic vessel activation in cancer. Ann N Y Acad Sci 979, 120-30 (2002).
11. Jussila, L. & Alitalo, K. Vascular growth factors and lymphangiogenesis. Physiol Rev 82, 673-700 (2002).
12. Carmeliet, P. Mechanisms of angiogenesis and arteriogenesis. Nat Med 6, 389-95. (2000).
13. Khurana, R. & Simons, M. Endothelial progenitor cells: precursors for angiogenesis. Semin Thorac Cardiovasc Surg 15, 250-8 (2003).
14. Witte, M.H., Bernas, M.J., Martin, C.P. & Witte, C.L. Lymphangiogenesis and lymphangiodysplasia: From molecular to clinical lymphology. Microsc Res Tech 55, 122-45. (2001).
15. Baldwin, M.E., Stacker, S.A. & Achen, M.G. Molecular control of lymphangiogenesis. Bioessays 24, 1030-40 (2002).
16. Jackson, D.G. The lymphatics revisited: new perspectives from the hyaluronan receptor LYVE-1. Trends Cardiovasc Med 13, 1-7 (2003).
17. Kriederman, B.M. et al. FOXC2 haploinsufficient mice are a model for human autosomal dominant lymphedema-distichiasis syndrome. Hum Mol Genet 12, 1179-85 (2003).
18. Ferrell, R.E. Research perspectives in inherited lymphatic disease. Ann N Y Acad Sci 979, 39-51; discussion 76-9 (2002).
19. Finegold, D.N. et al. Truncating mutations in FOXC2 cause multiple lymphedema syndromes. Hum Mol Genet 10, 1185-9 (2001).
20. Irrthum, A. et al. Mutations in the transcription factor gene SOX18 underlie recessive and dominant forms of hypotrichosis-lymphedema-telangiectasia. Am J Hum Genet 72, 1470-8 (2003).
21. Gale, N.W. et al. Angiopoietin-2 is required for postnatal angiogenesis and lymphatic patterning, and only the latter role is rescued by Angiopoietin-1. Dev Cell 3, 411-23. (2002).
22. Hirsch, N., Zimmerman, L.B. & Grainger, R.M. Xenopus, the next generation: X. tropicalis genetics and genomics. Dev Dyn 225, 422-33 (2002).
23. Klein, S.L. et al. Genetic and genomic tools for Xenopus research: The NIH Xenopus initiative. Dev Dyn 225, 384-91. (2002).
24. Khokha, M.K. et al. Techniques and probes for the study of Xenopus tropicalis development. Dev Dyn 225, 499-510. (2002).
25. Beck, C.W. & Slack, J.M. An amphibian with ambition: a new role for Xenopus in the 21 st century. Genome Biol 2(2001).
26. Chae, J., Zimmerman, L.B. & Grainger, R.M. Inducible control of tissue-specific transgene expression in Xenopus tropicalis transgenic lines. Mech Dev 117, 235-41. (2002).
27. Fu, L., Buchholz, D. & Shi, Y.B. Novel double promoter approach for identification of transgenic animals: A tool for in vivo analysis of gene function and development of gene-based therapies. Mol Reprod Dev 62, 470-6. (2002).
28. Eide, F.F., Eisenberg, S.R. & Sanders, T.A. Electroporation-mediated gene transfer in free-swimming embryonic Xenopus laevis. FEBS Lett 486, 29-32 (2000).
29. Jonak, J. Sperm-mediated preparation of transgenic Xenopus laevis and transmission of transgenic DNA to the next generation. Mol Reprod Dev 56, 298-300 (2000).
30. Kroll, K.L. & Kirschner, M.W. Easy passage: germline transgenesis in frogs. Proc Natl Acad Sci U S A 96,14189-90 (1999).
31. Fu, Y., Kan, D. & Evans, S. Transient transgenesis in Xenopus laevis facilitated by AAV-ITRs. Methods Mol Biol 127, 167-73 (1999).
32. Kroll, K.L. & Amaya, E. Transgenic Xenopus embryos from sperm nuclear transplantations reveal FGF signaling requirements during gastrulation. Development 122, 3173-83 (1996).
33. Yang, S., Lockwood, A., Hollett, P., Ford, R. & Kao, K. Overexpression of a novel Xenopus rel mRNA gene induces tumors in early embryos. J Biol Chem 273, 13746-52 (1998).
34. Nakano, H., Amemiya, S., Shiokawa, K. & Taira, M. RNA interference for the organizer-specific gene Xlim-1 in Xenopus embryos. Biochem Biophys Res Commun 274, 434-9 (2000).
35. Ohnuma, S., Mann, F., Boy, S., Perron, M. & Harris, W.A. Lipofection strategy for the study of Xenopus retinal development. Methods 28, 411-9 (2002).
36. Heasman, J. Morpholino oligos: making sense of antisense? Dev Biol 243, 209-14 (2002).
37. Iversen, P.L. Phosphorodiamidate morpholino oligomers: favorable properties for sequence-specific gene inactivation. Curr Opin Mol Ther 3, 235-8 (2001).
38. Corey, D.R. & Abrams, J.M. Morpholino antisense oligonucleotides: tools for investigating vertebrate development. Genome Biol 2, REVIEWS1015 (2001).
39. Ekker, S.C. Morphants: a new systematic vertebrate functional genomics approach. Yeast 17, 302-306 (2000).
40. Gatlin, J., Unett, D.J., Lerner, M.R. & Garcia, J.V. Efficient, long-term transgene expression in Xenopus laevis dermal melanophores. Pigment Cell Res 14, 275-82 (2001).
41. Cecconi, F. & Meyer, B.I. Gene trap: a way to identify novel genes and unravel their biological function. FEBS Lett 480, 63-71 (2000).
42. Hart, D.R. & Armstrong, J.B. Assessment of mutagenic damage by monofunctional alkylating agents and gamma radiation in haploid and diploid frogs, Xenopus laevis. Environ Mutagen 6, 719-35 (1984).
43. Bronchain, O.J., Hartley, K.O. & Amaya, E. A gene trap approach in Xenopus. Curr Biol 9, 1195-8 (1999).
44. Jin, S., McKee, T.D. & Oprian, D.D. An improved rhodopsin/EGFP fusion protein for use in the generation of transgenic Xenopus laevis. FEBS Lett 542, 142-6 (2003).
45. Hirsch, N. et al. Xenopus tropicalis transgenic lines and their use in the study of embryonic induction. Dev Dyn 225, 522-35 (2002).
46. Wallingford, J.B. Tumors in tadpoles: the Xenopus embryo as a model system for the study of tumorigenesis. Trends Genet 15, 385-8 (1999).
47. Olszewski, W. Regulation of water balance between blood and lymph in the frog, Rana pipiens. Lymphology 26, 154-5 (1993).
48. Koyama, T. & Horimoto, M. Lymphatic microcirculation in frog lung. Biorheology 25, 219-26 (1988).
49. Jones, J.M., Gamperl, A.K., Farrell, A.P. & Toews, D.P. Direct measurement of flow from the posterior lymph hearts of hydrated and dehydrated toads (Bufo marinus). J Exp Biol 200 ( Pt 11), 1695-702 (1997).
50. Garland, R.J. & Toews, D.P. Acid-base regulation in response to hypercapnia in the lymphatic and circulatory systems of the toad Bufo marinus. J Exp Biol 170, 271-6 (1992).
51. Liu, Z.Y. & Casley-Smith, J.R. The fine structure of the amphibian lymph sac. Lymphology 22, 31-5 (1989).
52. Ryu, H. et al. Frog lymph heart synthesizes and stores immunoreactive atrial natriuretic peptide. Gen Comp Endocrinol 87, 171-7 (1992).
53. Baldwin, A.L., Ferrer, P., Rozum, J.S. & Gore, R.W. Regulation of water balance between blood and lymph in the frog, Rana pipiens. Lymphology 26, 4-18 (1993).
54. Lametschwandtner, A., Aichhom, H. & Minnich, B. Current applications and methods of microvascular corrosion casting: a review. Micrscop. Microana/. 4 (suppl 2; proceedings)(1998).
55. Sive, H.L., Grainger, R.M. & Harland, R.M. Early Development of Xenopus laevis. A Laboratory Manual, (Cold Spring Harbor Laboratory Press, Cold Spring Harbor, New York, 2000).
56. Harland, R.M. In situ hybridization: an improved whole-mount method for Xenopus embryos. Methods Cell Biol 36, 685-95 (1991).
57. Newport, J. & Kirschner, M. A major developmental transition in early Xenopus embryos: I. characterization and timing of cellular changes at the midblastula stage. Cell 30(1982).
58. Nieuwkoop, P.D.F., J. Normal table of Xenopus laevis. Garand Publishing, New York (1994).
59. Heasman, J., Kofron, M. & Wylie, C. beta-catenin signaling activity dissected in the early Xenopus embryo: A novel antisense approach. Developmental Biology 222, 124-134 (2000).
60. Kroll, K.L. & Amaya, E. Transgenic Xenopus embryos from sperm nuclear transplantations reveal FGF signaling requirements during gastrulation. Development 122, 3173-3183 (1996).
61. Murray, A.W. Cell cycle extracts., 581-605 (Academic Press, Inc., San Diego, 1991).

## Claims

1. A transgenic tadpole **characterized in** having a lymphatic vessel system that is visualized by a reporter gene product expressed in said lymphatic vessel system, wherein said reporter gene is the transgene.

2. A transgenic tadpole according to claim 1 wherein said reporter gene is under control of a promoter specifically expressed in the lymphatic vessel system of said tadpole.

3. A transgenic tadpole according to claims 1-2 wherein said tadpole is a Xenopus tadpole.

4. A method to produce a transgenic tadpole according to claims 1-3 comprising introducing a vector comprising a reporter gene under control of a promoter specifically expressed in the lymphatic vascular system into cells of said tadpole.

5. A method according to claim 4 wherein said promoter is selected from the list comprising Podoplanin promoter, Prox-1 promoter, VEGFR-3 promoter and LYVE-1 promoter.

6. A method for visualizing the lymphatic vessel system in a tadpole comprising generating a transgenic tadpole comprising a reporter gene that is specifically expressed in the lymphatic vessel system.

7. Use of a transgenic tadpole according to claims 1-3 to identify a compound capable of modulating lymphatic vessel development comprising:
a) contacting said transgenic tadpole with a test compound,
b) comparing the lymphatic vessel system in said transgenic tadpole contacted with said test compound with the lymphatic vessel system of a transgenic tadpole that was not contacted with said test compound and,
c) determining the effect of said test compound on lymphatic vessel development, such that if lymphatic vessel development in the transgenic tadpole contacted with said test compound is different from the lymphatic vessel development in the transgenic tadpole that was not contacted with said test compound, said compound is a modulator of the lymphatic vessel system.

## Patentansprüche

1. Transgene Kaulquappe, **dadurch gekennzeichnet, dass** sie ein Lymphgefäßsystem aufweist, das mittels eines Reportergenprodukts sichtbar gemacht wird, das in dem Lymphgefäßsystem exprimiert wird, wobei es sich bei dem Reportergen um das Transgen handelt.

2. Transgene Kaulquappe nach Anspruch 1, wobei das Reportergen unter der Kontrolle eines Promotors steht, der spezifisch in dem Lymphgefäßsystem der Kaulquappe exprimiert wird.

3. Transgene Kaulquappe nach den Ansprüchen 1 - 2, wobei es sich bei der Kaulquappe um eine Xenopus-Kaulquappe handelt.

4. Verfahren zum Produzieren einer transgenen Kaulquappe nach den Ansprüchen 1 - 3, wobei das Verfahren das Einführen eines Vektors, der ein Reportergen unter der Kontrolle eines Promotors umfasst, der spezifisch in dem Lymphgefäßsystem exprimiert wird, in Zellen der Kaulquappe umfasst.

5. Verfahren nach Anspruch 4, wobei der Promotor aus der Liste ausgewählt ist, die Podoplanin-Promotor, Prox-1-Promotor, VEGFR-3-Promotor und LYVE-1-Promotor umfasst.

6. Verfahren zum Sichtbarmachen des Lymphgefäßsystems in einer Kaulquappe, wobei das Verfahren das Erzeugen einer transgenen Kaulquappe umfasst, die ein Reportergen umfasst, das spezifisch in dem Lymphgefäßsystem exprimiert wird.

7. Verwendung einer transgenen Kaulquappe nach den Ansprüchen 1 - 3 zum Identifizieren einer Verbindung, die die Lymphgefäßentwicklung modulieren kann, umfassend:
a) Inkontaktbringen der transgenen Kaulquappe mit einer Testverbindung,
b) Vergleichen des Lymphgefäßsystems in der transgenen Kaulquappe, die mit der Testverbindung in Kontakt gebracht wurde, mit dem Lymphgefäßsystem einer transgenen Kaulquappe, die nicht mit der Testverbindung in Kontakt gebracht wurde, und
c) Bestimmen der Auswirkung der Testverbindung auf die Lymphgefäßentwicklung, so dass, wenn die Lymphgefäßentwicklung in der transgenen Kaulquappe, die mit der Testverbindung in Kontakt gebracht wurde, sich von der Lymphgefäßentwicklung in der transgenen Kaulquappe, die nicht mit der Testverbindung in Kontakt gebracht wurde, unterscheidet, es sich bei der Verbindung um einen Modulator des Lymphgefäßsystems handelt.

## Revendications

1. Têtard transgénique **caractérisé en ce qu'**il possède un système de vaisseaux lymphatiques qui est visualisé par un produit de gène rapporteur exprimé dans ledit système de vaisseaux lymphatiques, dans lequel ledit gène rapporteur est le transgène.

2. Têtard transgénique selon la revendication 1, dans lequel ledit gène rapporteur est sous le contrôle d'un promoteur exprimé spécifiquement dans le système de vaisseaux lymphatiques dudit têtard.

3. Têtard transgénique selon les revendications 1 et 2, dans lequel ledit têtard est un têtard de xénopus.

4. Procédé de production d'un têtard transgénique selon les revendications 1 à 3 comprenant l'introduction d'un vecteur comprenant un gène rapporteur sous le contrôle d'un promoteur exprimé spécifiquement dans le système vasculaire lymphatique dans des cellules dudit têtard.

5. Procédé selon la revendication 4, dans lequel ledit promoteur est sélectionné parmi la liste comprenant un promoteur de podoplanine, un promoteur Prox-1, un promoteur VEGFR-3 et un promoteur LYVE-1.

6. Procédé de visualisation du système de vaisseaux lymphatiques chez un têtard comprenant la génération d'un têtard transgénique comprenant un gène rapporteur qui est exprimé spécifiquement dans le système de vaisseaux lymphatiques.

7. Utilisation d'un têtard transgénique selon les revendications 1 à 3 pour identifier un composé capable de moduler le développement de vaisseaux lymphatiques, comprenant :
a) la mise en contact dudit têtard transgénique avec un composé test,
b) la comparaison du système de vaisseaux lymphatiques chez ledit têtard transgénique mis en contact avec ledit composé test au système de vaisseaux lymphatiques d'un têtard transgénique qui n'a pas été mis en contact avec ledit composé test, et
c) la détermination de l'effet dudit composé test sur le développement de vaisseaux lymphatiques de sorte que, si le développement de vaisseaux lymphatiques chez le têtard transgénique mis en contact avec ledit composé test est différent du développement de vaisseaux lymphatiques chez le têtard transgénique qui n'a pas été mis en contact avec ledit composé test, ledit composé est un modulateur du système de vaisseaux lymphatiques.
